# EUROPÄISCHE PATENTSCHRIFT

(11) **EP 0 766 984 B1**
(45) Veröffentlichungstag und Bekanntmachung des Hinweises auf die Patenterteilung: **30.12.1998**
(21) Anmeldenummer: 96115817.7
(22) Anmeldetag: 02.10.1996
(51) Int. Cl.: B01D 9/00

(54) **Verfahren zur Stofftrennung aus einem flüssigen Gemisch durch Kristallisation**
Process for separating substances of a liquid mixture by crystallisation
Procédé de séparation des matières d'un mélange liquide par cristallisation

(30) Priorität: 02.10.1995 DE 19536792
(43) Veröffentlichungstag der Anmeldung: 09.04.1997
(73) Patentinhaber: BASF AKTIENGESELLSCHAFT, 67056 Ludwigshafen (DE)
(72) Erfinder: Eck, Bernd, 68519 Viernheim (DE); Maltry, Bernhard, 67283 Obrigheim (DE)
(74) Vertreter: Isenbruck, Günter, Dr.

(56) Entgegenhaltungen:
- US-A- 5 434 316

## Beschreibung

Die vorliegende Erfindung betrifft ein Verfahren zur Stofftrennung aus einem flüssigen Gemisch durch Kristallisation.

Die Kristallisation, insbesondere die fraktionierte Kristallisation, ist ein thermisches Trennverfahren, das bevorzugt Anwendung bei der Trennung flüssiger Stoffgemische findet. Besonders effektiv ist die Kristallisation bei der Trennung temperaturempfindlicher Stoffe, bei der Trennung azeotroper Gemische oder bei Stoffen mit niedrigen relativen Flüchtigkeiten.

Die Verfahren der fraktionierten Kristallisation beruhen auf der Bildung oder dem Wachstum von Kristallen an gekühlten Flächen. Hierbei wird das zu reinigende Stoffgemisch in den entsprechenden Kristallisator eingefüllt und anschließend an den gekühlten Flächen eine feste Phase ausgefroren, die sich in ihrer Zusammensetzung von der flüssigen Phase unterscheidet. Nachdem ein bestimmter Anteil des eingesetzten Stoffgemisches ausgefroren ist, wird die verbleibende flüssige Restphase abgetrennt. Üblicherweise erfolgt dies durch einfaches Abfließenlassen der Restphase. In DE-OS 26 06 364 ist ein Verfahren zur Stofftrennung aus einem flüssigen Gemisch durch fraktionierte Kristallisation beschrieben, bei dem man das flüssige Gemisch wiederholt in turbulenter Strömung durch eine indirekt gekühlte Kristallisationszone leitet mit der Maßgabe, daß die Kristallisationszone stets gefüllt ist. Eine Weiterentwicklung dieses Verfahrens beschreiben EP-B 0 279 439 und
DE-OS 37 08 709, bei dem die auf einer Kühlfläche ausgefrorenen Kristallschichten mit einer Reinigungslösung in Kontakt gebracht und damit gereinigt werden. In den beiden letztgenannten Druckschriften wird auch das Vorhandensein einer dünnen Impfkristallschicht auf den Kühlflächen offenbart, die durch einen kristallisierten Rieselfilm aus der vorangegangenen Kristallisationsstufe nach dem Entleeren der Schmelze entsteht. Zur Bildung dieser Impfkristallschicht ist nach Ablassen des Kristallisates in der vorangegangenen Kristallisationsstufe eine starke Unterkühlung erforderlich.

In DE-AS 17 69 123 wird beschrieben, daß die Kristallisation durch die Bildung lokaler Kristallisationszentren infolge starker Unterkühlung ausgelöst werden muß. Als weitere Variante wird das Auslösen der Kristallisation durch spontane Keimbildung infolge einer starken Unterkühlung eines dünnen Flüssigkeitsfilms, der aus der vorangegangenen Kristallisation stammt, angegeben. Es wird auch darauf hingewiesen, daß die spontane Keimbildung bei besonders tiefen Temperaturen des Kühlmittels erfolgen muß. Nachteilig ist hierbei insbesondere, daß zur Kühlung ein hoher energetischer Aufwand erforderlich ist.

Nachteilig bei der Unterkühlung ist, daß der gesamte Kristallisator abgekühlt werden muß. Nach dem Festfrieren der Impfschicht muß der gesamte Apparat wieder in die Nähe der Gleichgewichtstemperatur aufgeheizt werden, da infolge der zu großen Temperaturdifferenzen zwischen Impfschicht und Gleichgewichtstemperatur der zu reinigenden Schmelze unkontrolliertes Kristallwachstum zu Beginn der Kristallisation auftreten würde. Das Aufheizen des Apparats vor der Kristallisation bringt je nach vorausgegangener Unterkühlung große energetische Nachteile.

In der Patentschrift EP-B 0 218 545 wird darauf hingewiesen, daß bei der Kristallisation der Beginn der Kristallisation bei gewissen Materialien Probleme bieten kann, d.h. die Auslösung der Kristallbildung ist vielfach nicht reproduzierbar. Zur Lösung dieses Problems schlägt die Patentschrift vor, Einbauten in der Kristallisationsvorrichtung vorzusehen, die vorteilhaft mit Perforationen versehen sind, um zu Beginn der Kristallisation die Flüssigkeitsverteilung zu verbessern. Nachteilig ist hierbei neben dem zusätzlichen apparativen Aufwand der energetische Aufwand, der zum Kühlen und Heizen der Einbauten erforderlich ist.

Der Erfindung lag die Aufgabe zugrunde, die Verfahren zur Stofftrennung durch fraktionierte Kristallisation so zu modifizieren, daß gegenüber den bisher bekannten Verfahren deutliche Vorteile im Hinblick auf den Reinigungsaufwand und damit die Wirtschaftlichkeit der Reinigung erzielt werden.

Überraschenderweise wurde gefunden, daß sich dieses Problem dadurch lösen läßt, indem man vor der Kristallisation auf diejenigen Flächen des Kristallisators, von denen aus wahrend der Kristallisation Kristalle wachsen, eine zweiphasige Impfschicht, bestehend aus Schmelze oder Lösung und in dieser Schmelze oder Lösung suspendierten Kristallen des zu trennenden Gemisches, aufbringt.

Somit betrifft die Erfindung ein Verfahren zur Stofftrennung aus einem flüssigen Gemisch durch Kristallisation in einem Kristallisator, wobei man vor der Kristallisation auf diejenigen Flächen des Kristallisators, von denen aus während der Kristallisation Kristalle wachsen, eine zweiphasige Impfschicht in Form einer Suspension von einer Schmelze oder Lösung des zu trennenden Gemisches mit darin suspendierten Kristallen des Gemisches aufbringt. Bevorzugte Ausführungsformen des Verfahrens sind in den Unteransprüchen definiert.

Hierbei umfaßt gemäß der Erfindung der Begriff "Impfschichten" auch aus einer Suspension sedimentierte und auf eine Kristallisatorfläche festgefrorene Kristalle.

Die Erfindung bietet den Vorteil, daß zur Auslösung der Kristallisation der Impfschicht keine Unterkühlung der Schmelze oder Lösung mehr erforderlich ist. Damit wird ein unkontrolliertes Kristallwachstum, das leicht zu Einschlüssen von Verunreinigungen führt und somit den Reinigungseffekt herabsetzt, vermieden. Außerdem ist die Auslösung der Kristallisation in jedem Fall reproduzierbar. Daneben wurde gefunden, daß eine Schmelze oder Lösung mit suspendierten Kristallen die Flächen des Kristallisators besser benetzt als eine einphasige Impfschicht, wie sie in EP-B 0 218 545, DE-AS 17 69 123 und DE-OS 37 08 709 beschrieben ist. Die bessere Benetzung führt zu einer homogenen Belegung des Kristallisators mit einer Impfschicht und damit auch zu einer erheblich verbesserten Trennwirkung der Kristallisation. Somit wird die Reinigungswirkung der Kristallisation verbessert, ohne daß hierzu irgendwelche zusätzlichen Einbauten, wie in EP-B 0 218 545 vorgesehen, oder eine Unterkühlung der Schmelze und des Kristallisators, wie in DE-AS 17 69 123 beschrieben, was zu den oben genannten Nachteilen führt, erforderlich wären.

Die Durchführung des erfindungsgemäßen Kristallisationsverfahrens unterliegt an sich keiner Beschränküng. Grundsätzlich können alle Kristallisatoren verwendet werden, deren Funktion auf der Bildung von Kristallen auf gekühlten Flächen beruht. Das erfindungsgemäße Verfahren kann als dynamisches oder statisches Verfahren durchgeführt werden oder als Kombination dieser beiden Verfahren. Bei den statischen Verfahren, wie sie z.B. in US 3,597,164, EP 0 323 377 und FR 2 668 946 beschrieben sind, wird die flüssige Phase nur durch freie Konvektion bewegt, während bei den dynamischen Verfahren die Kristallisation bei einer erzwungenen Konvektion der flüssigen Phase durchgeführt wird. Dies kann durch eine erzwungene Strömung in volldurchströmten Wärmeüberträgem erfolgen, wie z.B. in DE 26 06 364 beschrieben, oder durch die Aufgabe eines Rieselfilms auf eine gekühlte Wand erfolgen, wie es z.B. DE-AS 17 69 123 und EP-B 0 218 545 beschreiben.

Das erfindungsgemäße Verfahren ist sowohl für die Kristallisation aus der Schmelze der abzutrennenden Substanz als auch aus einer Lösung der abzutrennenden Substanz in einem Lösungsmittel anwendbar und eignet sich allgemein für alle Substanzen, die sich auf einer gekühlten Fläche auskristallisieren lassen. Besondere technische Bedeutung hat die fraktionierte Kristallisation aus der Schmelze erlangt. Besonders zur Trennung geeignete flüssige Gemische sind solche mit einem Schmelzpunkt von -50°C bis +300°C, die sich bei den angewandten Temperaturen nicht zersetzen und vorzugsweise z.B. N-Vinylpyrrolidon, Naphthalin, Anthracen, Bisphenol A, Benzoesäure, Monochloressigsäure, Acrylsäure, Methacrylsäure, Piperazin, Caprolactam, Xylol (Isomerentrennung), Hexamethylendiamin, Toluoldiisocyanate (Isomerentrennung) und Diphenylmethandiisocyanat (Methylendi(phenylisocyanat); Isomerentrennung) enthalten.

Die in dem erfindungsgemäßen Verfahren verwendete Suspension kann in unterschiedlichen Apparaten oder Vorrichtungen erzeugt werden. In einer bevorzugten Ausführungsform der Erfindung friert man aus einer Schmelze oder Lösung des zu trennenden Gemisches Kristalle aus und bringt diese in die Schmelze oder Lösung ein. Bevorzugt werden in sogenannten Kratzkühlem oder Rührkesseln insbesondere mit wandgängigen Rührern durch indirekte Kühlung Kristalle ausgefroren, die mit Hilfe von Schabelementen von den gekühlten Wänden in die Suspension gefördert werden. Daneben besteht auch die Möglichkeit, durch Abkühlen der Schmelze oder Lösung entweder über den Kristallisator selbst oder über in den Kristallisator oder andere Apparate eingebaute kühlbare Elemente (z.B. Kühlfinger, Kühlstrecken oder Rührbehälter) Kristalle direkt in der Schmelze oder Lösung zu erzeugen und auf diese Weise eine Suspension zu erzeugen. Dies hat den Vorteil, daß die Kristalle nicht abgeschabt werden müssen. Die Verwendung kühlbarer Elemente ist vorteilhaft, da nicht der gesamte Kristallisator abgekühlt werden muß. Es ist auch möglich, daß man im Kristallisator oder außerhalb von diesem eine Suspension erzeugt und die Kristalle aus der Suspension im Kristallisator auf die Kristallisationsflächen sedimentieren läßt, wo sie als Impfkristalle wirken. Der Feststoffgehalt der Suspension liegt zwischen 0 g Feststoff/g Suspension und 60 g Feststoff/g Suspension.

In einer bevorzugten Ausgestaltung der Erfindung bringt man die Suspension auf die Kristallisatorflächen auf, indem man den Kristallisator mit der Suspension füllt und anschließend entleert. Nach der Entleerung verbleibt eine Suspensionsschicht auf den Kristallisatorflächen, die dann (bei ihrer Gleichgewichtstemperatur) angefroren wird. Entsprechend kann verfahren werden, wenn die Suspension im Kristallisator selbst erzeugt wird. Daneben besteht auch die Möglichkeit, die Suspension über übliche Verteilervorrichtungen, wie Überlaufwehre, Düsen oder Spaltverteiler, auf die Kristallisatorflächen aufzubringen. Dadurch kann ein Befüllen des Apparates mit anschließendem Entleeren vermieden werden.

Erfindungsgemäß geeignete Verfahren zur Durchführung der Kristallisation sind z.B. in US 5,329,021, DE 26 06 364, DE 17 69 123 und EP 0 475 893 beschrieben. Erfindungsgemäß wird nach Aufbringen der Impfschicht das zu reinigende Stoffgemisch als Schmelze oder Lösung in den Kristallisator eingefüllt und anschließend an den gekühlten Flächen des Kristallisators eine feste Kristallisatphase ausgefroren. Da bereits zu Beginn der Kristallisationsschritte eine kristalline Phase als Impfschicht vorliegt, muß das zu reinigende Stoffgemisch zur Kristallisatbildung nicht mehr unterkühlt werden. Beim Befüllen des Kristallisators ist die zu reinigende Schmelze oder Lösung entweder auf Schmelztemperatur oder wenige Grad Celsius über der Schmelztemperatur, um eine Kristallbildung in den Zuführleitungen zu vermeiden. Werden die Kristalle aus der Suspension aussedimentiert und festgefroren, bedarf es keines Entleerens und anschließenden Füllens mit Rohschmelze oder -lösung mehr. In diesem Fall kann sofort kristallisiert werden.

Nach Ausfrieren eines bestimmten Anteils des eingesetzten Stoffgemisches trennt man die beiden Phasen voneinander, was z.B. durch einfaches Abfließenlassen oder Abpumpen der flüssigen Rückstandsphase erfolgen kann. Anschließend erwärmt man die abgetrennte kristalline Phase auf eine Temperatur, bei der sich die Kristalle verflüssigen und unterwirft diese Schmelze oder Lösung ggf. weiteren Kristallisationsschritten. Ebenso kann die Rückstandsphase weiteren Kristallisationsschritten unterworfen werden, wie es weiter unten näher beschrieben ist. Der Kristallisation können sich weitere Reinigungsschritte anschließen. Besonders geeignet sind ein Waschen der Kristallschicht mit Reinigungsflüssigkeit, wie es z.B. in DE-OS 37 08 709 beschrieben ist, und/oder die Durchführung eines sogenannten Schwitzens der Kristallschicht. Beim Schwitzen wird die Temperatur der Kristallschicht angehoben, wobei bevorzugt die höher verunreinigten Bereiche der Kristallschicht abschmelzen und so eine zusätzliche Reinigungswirkung erzielt wird.

Das erfindungsgemäße Verfahren wird in einer oder mehreren Kristallisationsstufen durchgeführt. Allgemein kann man die Kristallisationsstufen in Reinigungsstufen und Abtriebsstufen einteilen. Zur Erhöhung der Trennwirkung können sich einer Kristallisationsstufe weitere Reinigungs-(Kristallisations-)stufen anschließen, in denen jeweils das Kristallisat der vorhergehenden Stufe kristallisiert wird. Zur Erhöhung der Ausbeute des Verfahrens können sogenannte Abtriebsstufen vorgesehen sein, in denen die flüssige Rückstandsphase Abtriebs-(Kristallisations)stufen unterzogen wird. Vorzugsweise wird hierbei nach dem Gegenstromprinzip vorgegangen, bei dem die Kristallisatströme den Stufen mit der nächsthöheren Stufennummer und die Kristallisationsrückstandsströme den Stufen mit der nächstniedrigeren Stufennummer zugeführt werden. Die Anzahl der Kristallisationsstufen und damit auch der Reinigungs- und Abtriebsstufen hängt von der Trennaufgabe ab und kann vom Fachmann im Rahmen üblicher Versuche ermittelt werden.

Prinzipiell kann zur Erzeugung der Suspension jede im Prozeß auftretende Zusammensetzung der Schmelze oder Lösung verwendet werden. In einer bevorzugten Ausführungsform der Erfindung wird zur Erzeugung der Suspension eine Schmelze oder Lösung verwendet, die reiner als die in der jeweiligen Stufe zu kristallisierende Schmelze oder Lösung ist. Vorzugsweise wird das die jeweilige Kristallisationsstufe verlassende geschmolzene Kristallisat verwendet.

Die Erfindung wird anhand des folgenden Beispiels, das eine bevorzugte Ausführungsform der Erfindung darstellt, näher erläutert.

### Beispiel

N-Vinylpyrrolidon mit einer Ausgangsverunreinigung von 0,6 Gew.-% (bezogen auf 100 Gew.-% N-Vinylpyrrolidon (diese Verunreinigungskonzentration entspricht der im US-Patent US-A-5 329 021 genannten Verunreinigungskonzentration)) wurde in einem Kristallisator, wie er in der Offenlegungsschrift DE-A-26 06 364 (BASF) beschrieben ist, in zwei Reinigungsstufen und vier Abtriebsstufen kristallisiert. Hierbei wurde in allen Kristallisationsstufen als Impfschicht auf die Flächen des Kristallisators eine zweiphasige Suspension bestehend aus N-Vinylpyrrolidonschmelze mit suspendierten N-Vinylpyrrolidon-Kristallen aufgebracht. Zum Erzeugen der Impfschicht wurde der Kristallisator mit der in der jeweiligen Stufe zu reinigenden Schmelze gefüllt und durch Abkühlen des Apparats eine Suspension erzeugt. Danach wurde der Apparat entleert und die auf den Kristallisatorflächen verbleibende Suspension festgefroren. Die Gleichgewichtstemperaturen der Schmelzen während der Kristallisation in den einzelnen Stufen waren wie folgt:
- Reinigungsstufe 5:: 13,5 bis 12,9°C
- Reinigungsstufe 6:: 13,8 bis 13,6°C
- Abtriebsstufe 1:: 13,1 bis 12,6°C
- Abtriebsstufe 2:: 12,5 bis 11,4°C
- Abtriebsstufe 3:: 11,2 bis 8,5°C
- Abtriebsstufe 4:: 8,5 bis 4,3°C

Bis auf die Abtriebsstufe 4, die statisch gefahren wurde, wurden alle anderen Stufen dynamisch gefahren. Das Verhältnis der in einer Stufe ausgefrorenen Kristallmasse zur der in dieser Stufe eingesetzten Kristallmasse betrug 0,8 in der ersten Reinigungsstufe und 0,75 in der zweiten Reinigungsstufe. In der ersten Reinigungsstufe wurde eine Verunreinigungskonzentration von 0,102 Gew.-% (bezogen auf 100 Gew.-% N-Vinylpyrrolidon) im Kristallisat erreicht. Gegenüber dem US-Patent entspricht dies einer um den Faktor 4 niedrigeren Verunreinigungskonzentration (dort betrug die Verunreinigungskonzentration 0,4 Gew.-% (bezogen auf 100 Gew.-% N-Vinylpyrrolidon)). Das Kristallisat wird danach einer zweiten Reinigungsstufe zugeführt und verläßt diese mit einer Verunreinigungskonzentration von 190 ppm, was, gegenüber dem US-Patent, in dem die Verunreinigungskonzentration 500 ppm betrug, eine etwa um den Faktor 2,5 niedrigere Verunreinigungskonzentration bedeutet.

Im Vergleich zu bekannten Verfahren wird somit mit dem erfindungsgemäßen Verfahren durch die Aufbringung einer Impfschicht aus N-Vinylpyrrolidon vor der jeweiligen Kristallisation bei gleichen Ausgangsbedingungen, gleicher Zahl der Reinigungsstufen und gleicher ausgefrorener Kristallmasse bezogen auf die eingesetzte Schmelze oder lösung, eine um den Faktor 2,5 niedrigere Verunreinigungskonzentration im gereinigten N-Vinylpyrrolidon erzielt. Bei gleicher Produktspezifikation bedeutet dies eine Einsparung von Reinigungsstufen und damit eine erhebliche Reduzierung des erforderlichen Trennaufwands.

## Patentansprüche

1. Verfahren zur Stofftrennung aus einem flüssigen Gemisch durch Kristallisation in einem Kristallisator, dadurch gekennzeichnet, daß man vor der Kristallisation auf diejenigen Flächen des Kristallisators, von denen aus während der Kristallisation Kristalle wachsen, eine zweiphasige Impfschicht in Form einer Suspension von einer Schmelze oder Lösung des zu trennenden Gemisches mit darin suspendierten Kristallen des Gemisches aufbringt.

2. Verfahren nach Anspruch 1, dadurch gekennzeichnet, daß man die Suspension erzeugt, indem man aus einer Schmelze oder Lösung des zu trennenden Gemisches Kristalle ausfriert und diese in die Schmelze oder Lösung einbringt.

3. Verfahren nach Anspruch 1 oder 2, dadurch gekennzeichnet, daß man die Impfschicht aufbringt, indem man die Suspension in den Kristallisator füllt oder die Suspension im Kristallisator selbst erzeugt, anschließend den Kristallisator entleert und die auf den Kristallisatorflächen verbleibende Suspensionsschicht anfriert.

4. Verfahren nach Anspruch 1 oder 2, dadurch gekennzeichnet, daß man die Impfschicht aufbringt, indem man die Suspension über Verteilervorrichtungen auf die Kristallisatorflächen aufbringt.

5. Verfahren nach einem der vorhergehenden Ansprüche, dadurch gekennzeichnet, daß man die Kristallisation in einer oder mehreren Kristallisationsstufen durchführt.

6. Verfahren nach Anspruch 5, dadurch gekennzeichnet, daß man bei Durchführung der Kristallisation in mindestens zwei Stufen zur Erzeugung der Suspension eine Schmelze oder Lösung verwendet, die reiner als die in der jeweiligen Stufe zu kristallisierende Schmelze oder Lösung ist.

7. Verfahren nach einem der vorhergehenden Ansprüche, dadurch gekennzeichnet, daß man die Kristallisation statisch oder dynamisch durchführt.

8. Verfahren nach einem der vorhergehenden Ansprüche, dadurch gekennzeichnet, daß man flüssige Gemische mit einem Schmelzpunkt von -50°C bis +300 °C, vorzugsweise enthaltend die Verbindungen N-Vinylpyrrolidon, Naphthalin, Anthracen, Bisphenol A, Benzoesäure, Monochloressigsäure, Acrylsäure, Methacrylsäure, Piperazin, Caprolactam, Xylol, Toluoldiisocyanat, Diphenylmethandiisocyanat oder Hexamethylendiamin, einsetzt.

## Claims

1. A process for separating substances from a liquid mixture by crystallization in a crystallizer, wherein a two-phase seed layer in the form of a suspension of a melt or solution of the mixture to be separated, with crystals of the mixture suspended therein, is applied, prior to the crystallization, to those surfaces of the crystallizer from which crystals grow during the crystallization.

2. A process as claimed in claim 1, wherein the suspension is produced by freezing crystals out of a melt or solution of the mixture to be separated and introducing these crystals into the melt or solution.

3. A process as claimed in claim 1 or 2, wherein the seed layer is applied by introducing the suspension into the crystallizer or producing the suspension in the crystallizer itself, then emptying the crystallizer and freezing the suspension layer remaining on the crystallizer surfaces.

4. A process as claimed in claim 1 or 2, wherein the seed layer is applied by applying the suspension to the crystallizer surfaces by means of distributor apparatuses.

5. A process as claimed in any of the preceding claims, wherein the crystallization is carried out in one or more crystallization stages.

6. A process as claimed in claim 5, wherein, when the crystallization is carried out in at least two stages, a melt or solution which is purer than the melt or solution to be crystallized in the particular stage is used for producing the suspension.

7. A process as claimed in any of the preceding claims, wherein the crystallization is carried out statically or dynamically.

8. A process as claimed in any of the preceding claims, wherein liquid mixtures having a melting point of from -50 to +300°C, preferably containing the compounds N-vinylpyrrolidone, naphthalene, anthracene, bisphenol A, benzoic acid, monochloroacetic acid, acrylic acid, methacrylic acid, piperazine, caprolactam, xylene, toluene diisocyanate, diphenylmethane diisocyanate or hexamethylenediamine, are used.

## Revendications

1. Procédé de séparation des matières contenues dans un mélange liquide, par cristallisation dans un cristallisoir, caractérisé en ce qu'avant la cristallisation, on applique sur les surfaces du cristallisoir à partir desquelles des cristaux croissent pendant la cristallisation une couche biphasique d'inoculation sous la forme d'une suspension d'un bain de fusion ou d'une solution du mélange à séparer, dans laquelle des cristaux du mélange sont en suspension.

2. Procédé selon la revendication 1, caractérisé en ce que l'on crée la suspension en gelant des cristaux d'un bain de fusion ou d'une solution du mélange à séparer, et qu'on les apporte dans le bain de fusion ou la solution.

3. Procédé selon les revendications 1 ou 2, caractérisé en ce que l'on applique la couche d'inoculation en introduisant la suspension dans le cristallisoir ou en créant la suspension dans le cristallisoir lui-même, en vidant ensuite le cristallisoir et en gelant la couche de suspension restant sur les surfaces du cristallisoir.

4. Procédé selon les revendications 1 ou 2, caractérisé en ce que l'on applique la couche d'inoculation en appliquant la suspension sur les surfaces du cristallisoir à l'aide de dispositifs de répartition.

5. Procédé selon l'une des revendications précédentes, caractérisé en ce que l'on réalise la cristallisation en une ou plusieurs étapes de cristallisation.

6. Procédé selon la revendication 5, caractérisé en ce que lorsque l'on exécute la cristallisation en au moins deux étapes, on utilise pour créer la suspension un bain de fusion ou une solution qui sont plus purs que le bain de fusion ou la solution à cristalliser dans chaque étape.

7. Procédé selon l'une des revendications précédentes, caractérisé en en ce que l'on réalise la cristallisation par voie statique ou par voie dynamique.

8. Procédé selon l'une des revendications précédentes, caractérisé en ce que l'on utilise des mélanges liquides dont le point de fusion est compris entre - 50° C et + 300° C, qui contiennent de préférence les composés N-vinylpyrrolidone, naphtaline, anthracène, bisphénol A, acide benzoïque, acide monochloroacétique, acide acrylique, acide méthacrylique, pipérazine, caprolactame, xylène, diisocyanate de toluène diisocyanate de diphénylméthane ou hexaméthylènediamine.
